(19)

**Europäisches Patentamt**
**European Patent Office**
**Office européen des brevets**

(11) **EP 3 722 255 A1**

(12) **EUROPEAN PATENT APPLICATION**

(43) Date of publication:
**14.10.2020 Bulletin 2020/42**

(51) Int Cl.:
***B82Y 5/00*** (2011.01)

(21) Application number: **19382271.5**

(22) Date of filing: **11.04.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Universitat Autònoma de Barcelona**
  **08193 Bellaterra (Barcelona) (ES)**
• **Institut Recerca Hospital de la Santa Creu I Sant Pau**
  **08025 Barcelona (ES)**

(72) Inventors:
• **VILLAVERDE CORRALES, ANTONIO**
  **08320 El Masnou (Barcelona) (ES)**
• **VÁZQUEZ GÓMEZ, ESTHER**
  **08320 El Masnou (Barcelona) (ES)**
• **LÓPEZ LAGUNA, Héctor**
  **08915 Badalona (Barcelona) (ES)**
• **SÁNCHEZ, Julieta María**
  **08210 Barberà del Vallès (Barcelona) (ES)**
• **MANGUES BAFALLUY, Ramón**
  **08005 Barcelona (Barcelona) (ES)**

(54) **PROTEIN NANO- OR MICROPARTICLES AS ARTIFICIAL INCLUSION BODIES**

(57) The invention relates to protein particles comprising a cluster of one or more types of assembled self-contained protein, wherein the particle has a size from 50 nm to 50 micrometers; is in form of a pellet in aqueous media; is mechanically stable; and it release a particular percentage by weight of the self-contained protein within a predetermined period of time, and any other compound contained in the particle. Particular methods for obtaining the particles are also disclosed, said methods comprising the addition of salts to allow precipitation of proteins. Particular protein particles comprising lipids associated with the assembly of self-contained proteins are also disclosed. The invention also relates to several uses of the particle, in particular medical uses and to pharmaceutical and cosmetic compositions comprising the particles.

EP 3 722 255 A1

**Description**

**Technical Field**

Present invention relates to the field of self-assembled biological molecules and to particular uses of the same in the field of medicine.

**Background Art**

Bacterial inclusion bodies (IBs) are mechanically stable, insoluble, discrete, and particulate proteinaceous materials produced in recombinant bacteria, with particle sizes ranging from aprox. 50 to 1500 nm, and with shapes including cylindrical, amorphous, spherical or ellipsoid. They contain one or few functional protein species (together with other possible components) that can be released under physiological conditions. They commonly occur in the cytoplasm of recombinant bacteria, as a consequence of conformational stresses occurring during protein overproduction. They are mainly formed by the recombinant protein, but also contain variable but uncharacterized amounts of bacterial proteins, lipids carbohydrates and nucleic acids (see Neubauer, et al., "Protein inclusion bodies in recombinant bacteria", 237-292, Springer- 2006). IBs have been traditionally observed as an obstacle to obtain functional protein by recombinant procedures, since insoluble protein was believed to be unfolded and inactive. However, in 2005, inventors and others discovered that IBs are formed by a mixture of non-functional and functional polypeptides, and that IBs are protein particles with biological activity, usable in biotechnology and biomedicine (see. Garcia-Fruitos, E. et al. "Aggregation as bacterial inclusion bodies does not imply inactivation of enzymes and fluorescent proteins", Microbial cell factories2005, vol. no. 4, 27, doi:10.1186/1475-2859-4-27; and Gonzalez-Montalban, N., et al., "Recombinant protein solubility - does more mean better?", Nature biotechnology 2007, vol. no. 25, pp.:718-720, doi:10.1038/nbt0707-718). The non-functional protein has an amyloidal structure that corresponds to around 20-40 % of the bulk material. The functional protein is embedded in this amyloidal structure in a sort of sponge-like organization.

Thus, IBs are mechanically stable functional materials that are nontoxic when exposed to cells or to living beings, through oral administration or injection. Because of the combination of mechanical stability and functionality, IBs are then explored as self-immobilized catalysts, showing promises in biotechnological industries and applications (see Rinas, U. et al. "Bacterial Inclusion Bodies: Discovering Their Better Half". Trends in biochemical sciences, doi:10.1016/j.tibs.2017.01.005 (2017); and de Marco, A. et al.

de Marco, A."Bacterial inclusion bodies are industrially exploitable amyloids", FEMS microbiology reviews, doi:10.1093/femsre/fuy038 (2018)).

As indicated, when in contact with mammalian cells, IBs tend to penetrate them, apparently in absence of toxicity, and are able to release the embedded functional protein with full biological activity. The cell contact and penetration can be in addition targeted by fusing a cell-targeting peptide to the recombinant IB protein. Functional IBs can then act as NANOPILLS for the delivery of therapeutic proteins. This principle has been demonstrated for chaperones, enzymes, growth factors and structural proteins, and it can be also applied to cytokines, hormones, and any functional protein having a physiological role, whose activity needs to be restored or enhanced. This can be also used by *de novo* designed proteins with activities or combination of them, not present in nature. When administered by local injection in tumor or subcutaneously, IBs are stable in the injection site and slowly release the IB protein for a functional effect, either locally or remotely, when the protein is targeted with a homing peptide. When the IB protein self-assembled as tumor-targeted nanoparticles, they released from IBs in the assembled form, both *in vitro* and *in vivo*. Then, subcutaneous injection in a remote place provides a long-lasting depot platform for the delivery of tissue-targeted therapeutic protein nanoparticles for diverse clinical applications.

As catalysts, IBs do not pose any regulatory issues and are highly convenient. However, clinical applicability of IBs is not exempt of drawbacks and for this reason it is restricted by the following facts:

First, IBs have a bacterial origin, and they contain irremovable bacterial components at variable composition such as cell wall, nucleic acids, endotoxins and undesired proteins, incompatible with a drug formulation. Moreover, due to the cell factory base, IBs carry on with several homogeneity issues between manufacturing batches.

Secondly, proteins that glycosylate or that follow other post-translational modifications not done by bacterial cells cannot be produced as functional IBs.

Finally, and as previously enunciated, although apparently non-toxic even having an amyloid structure, few assays and data are still in relation thereto.

For all these reasons, the provision of alternative structures for delivering compounds of interest (small molecule drugs, therapeutic proteins, enzymes, etc.) in cells of organisms is evidence, but the maintenance of all those beneficial behaviour and features of IBs is also a desire. In particular, their high penetration to cells and the mechanic stability.

## Summary of Invention

Inventors developed insoluble protein particles, with a slow release profile of the proteins at physiological conditions comprising clusters or groupings of assembled self-contained proteins that surprisingly mimic the goodness of IBs but that are exempts of the drawbacks. Artificial IBs have been prepared *in vitro* (cell-free engineered) without the presence of bacterial cells, thus in a fully synthetic mode. According to the best of inventor's knowledge this is the first time protein micro and nanoparticles have been done resembling the natural IBs, avoiding furthermore by this way the problematic features existing in natural IBs as described before.

Thus, a first aspect of the invention is a protein nano- or microparticle comprising a cluster of one or more types of assembled self-contained proteins, wherein the nano-or micro particle:

-    has a size, measured as hydrodynamic diameter, from 50 nanometers (nm) to 50 micrometers ($\mu$m);
-    is mechanically stable, which means that the cluster of self-contained proteins remains structured when submitted at sonication conditions including 5 rounds of 40 seconds; 0.5 of pulse on; 0.5 of pulse off and a wave width of 10 % in a high intensity sonicator Branson sonifer 450,with 3 mm-diameter titanium probe;
-    is in the form of a precipitated pellet in aqueous media, when centrifuged at 15.000 g at a temperature from 4 °C to 30 °C; and
-    it releases an amount of assembled self-contained proteins lower than 50 % by weight in relation to the total weight of assembled self-contained protein within 24 hours and when resuspended in an aqueous media at physiological temperature.

Thus, the nanoparticle or microparticle is obtained as an insoluble pellet, said solubility measured at a temperature from 4 °C to 30 °C (or at room temperature from 18 °C to 30°C) in an aqueous media.

Sonifier used to test mechanical stability transforms altern current (AC) high voltage to 20 kHz of electrical energy. This high-frequency energy is fed to a converter to obtain mechanical vibration.

Thus, there has been developed synthetic or cell-free engineered protein nano- or microparticles that behave as IBs. For synthetic or cell-free engineered is to be understood that no prokaryotic or eukaryotic cells are the providers/suppliers of said nano- and/or microparticles, but that they are synthesized in a recipient. The cluster or grouping of one or more types of assembled self-contained proteins are indeed configuring a protein scaffold. It is a tridimensional scaffold in which other compounds different from the self -contained proteins can be adhered or embedded between interstitial spaces defined by the assembled proteins, like a net in which other components can be entrapped, and/or adsorbed. This scaffold remains structured once submitted to sonication conditions.

Therefore, the protein nano- or microparticle are free of prokaryotic or eukaryotic cell components different from the self-contained proteins.

As will be illustrated in Examples below, the protein particles of the invention do mimic the properties of natural IBs. Namely, they are mechanically stable; they have a size from 50 nm to 50 $\mu$m; they are formed by proteins that are self-contained and they optionally comprise additional functional proteins. In certain particular embodiments they are formed by one or more protein species (obtained from an external source, such as a protein supplier), plus any necessary additional components (such as lipids) at a defined proportion. They allow the release of the proteins, either the ones assembled and self-contained or any other protein that is embedded in the cluster of assembled self-contained proteins; and they penetrate mammalian cells in absence of toxicity and release, intracellularly, functional protein. In addition, cell penetrability is targetable.

The design and fabrication of artificial IBs has been approached in the laboratory by means of biophysical proteomic methods as described in the detailed description.

Thus, a second aspect of the invention is a method for the synthesis of a protein nano- or microparticle as defined above, wherein the method comprises the following steps:

(a) mixing in a recipient one or more types of proteins with a polar solvent;
(b) submitting the mixture of step (a) to protein assembly conditions to obtain a cluster of assembled self-contained proteins; and
(c) isolating the clusters.

This second aspect is thus a cell-free method for the synthesis of a protein nano- or microparticle.

The invention also relates to films comprising one or more lipids and one or more denatured proteins, which are self-assembled and configure a tridimensional cluster or net, said film disposed on a support.

As will be illustrated in the examples below, the protein nano- or microparticles of the invention penetrate into tumor cells. Thus, a third aspect of the invention is a protein nano-or microparticle as defined above for use as a medicament.

These protein nano- or microparticles resembling natural inclusion bodies are thus, synthetic inclusion bodies, and it is another aspect of the invention a protein nano- or microparticle as defined above as synthetic bacterial inclusion body.

The protein nano- or microparticles comprising functional proteins may, in addition, be used as subcutaneous implants. By this way they can deliver to organisms the compounds with therapeutic effect. These compounds are either the self-contained proteins configuring the cluster of the particle that is sustainly solubilized or decomposed within a period of time, said self-contained proteins having a therapeutic effect, or any therapeutic agent different of the protein that can be embedded or adhered to the cluster of the particles or even linked to the self-contained proteins with an hydrolysable bond. All these options are disclosed in more detail below.

Therefore, another aspect of the invention is a drug-delivery system comprising the protein nano- or microparticles as defined above. For "drug" is to be understood any compound or even composition with a proved therapeutic effect.

The protein nano- or microparticles of the invention can be used as actives in pharmaceutical compositions and so, it is also an aspect of the invention a pharmaceutical composition comprising a therapeutically effective amount of the protein nano- or microparticles disclosed above together with pharmaceutically acceptable excipients or carriers.

Also depending on the nature and functionality (or activity) of the proteins, the protein nano- or microparticles of the invention can be used as actives in cosmetic compositions and so, it is also an aspect of the invention a cosmetic composition comprising a cosmetic effective amount of the protein nano- or microparticles disclosed above together with one or more appropriate cosmetically acceptable excipients or carriers.

**Brief Description of Drawings**

FIG. 1, related with Example 2, is a graphic with the detected diameter (calculated by DLS) and the relative abundance of these diameters (% volume) of the protein nano- or microparticles of the invention.

FIG. 2, related with Example 2, shows Field Emission Scanning Electron Microscopy (FESEM) images of synthetic IBs of the invention. Electron high tension, (EHT) = 1.00 kV; working distance (WD) = 3.3 mm; Signal A = secondary electron (SE2); Mag = 2.56 K X.

FIG. 3, related with Example 3, shows fluorescence emission relative intensity measured in a Cary Eclipse spectrofluorimeter (Agilent Technologies, Mulgrave, Australia), at a wavelength of 512 nm] of different concentrations of functional T22-GFP-H6 (g/l) synthetic IBs (light circles) of Example 1. As control nanoparticles (NPs; dark circles) of T22-GFP-H6 formed through assembly of the GFP-containing monomers produced in *E. coli.*

FIG. 4, related with Example 4, is a graph with the amount of released functional protein in % (i.e. release of functional fluorescent T22-GFP-H6) along time (Time in hours (h)).

FIG. 5 is a graph with the detected intracellular fluorescence in HeLa cells exposed to different entities, analyzed by flow cytometry at different time points. Different assays of internalization were performed using different entities for internalization.

FIG. 6 is a graphic with the percentage of cell viability (% cell viability) at 24 (left column), 48 (middle column) and 72 (right column) hours of cells (HeLa) cultured with the presence of different entities.

FIG. 7, related with Example 7, includes FESEM images of several protein microparticles obtained by assembly of the fused protein T22-GFP-H6 with a salt of divalent cations at different salt:protein molecular proportions (images 1, 2 and 3 for proportions 40:1, 100:1 and 150:1, respectively). They are taken in comparison with a natural inclusion body produced directly in bacteria (image 0).

**Detailed description of the invention**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

The term "self-contained protein" relates to proteins that when assembled with other proteins constitute by themselves a free-standing unit (cluster) or that remain configuring a certainly ordered scaffold or structure or grouping made of proteins. The term "cluster" relates such to a discrete grouping of proteins, and additionally of other compounds.

For "Live cell-free engineered" or "cell-free manufactured" is to be understood that the microparticles and nanoparticles are synthetically obtained, free of any live cell acting as source of said particles. Thus, the proteins forming the cluster although of live prokaryotic or eukaryotic cells origin, they are not assembled inside any cell or in the presence of cells within the recipient where assembly is performed. Thus, the particles are free of any compounds that could be present in case the particles where formed in live prokaryotic or eukaryotic cells (cell debris, cytoplasmic components, and membrane components).

The term "assembling agent" relates to any compound and/or physical condition allowing certain molecules, such as proteins or a mixture of proteins and lipids, to configure discrete units or groupings of molecules (in a mode of tridimensional scaffolds). Depending on the assembling agent or conditions and of the nature of the proteins, said groupings can be organized or unorganized groupings.

The term "release of an amount of assembled self-contained proteins lower than 50 % by weight in relation to the total weight of assembled self-contained protein", means that the microparticle or nanoparticle is indeed a delivery system with a slow release profile, and the cluster configured by assembled proteins loses less than 50% w/w of the self-contained proteins over a period of time and at physiological conditions. Physiological conditions include a temperature from 34.5°C to 42°C (being normal from 36.5 °C to 37.5°C (the said physiological temperature) and pH around 7 (6.5-7.8). This release is also to be understood as a mode of delivering said proteins in a particular media. The media can be, in particular, a tissue from a living organism in such a way that the particle is finally decomposed, in particular in a sustained way.

The term "insoluble pellet" means that when manufactured in aqueous media and at a temperature from 4 °C to 30 °C (or at room temperature from 18 °C to 30°C), the nano- or microparticles precipitate or they sediment at the bottom of the recipient where they are formed. Thus, the nano-or microparticles are in form of a "precipitated pellet" in the aqueous media where they were manufactured (at 4°C-30 °C) and that is visible among a predetermined period of time allowing sedimentation, or by means of mild centrifugation, for example at 15.000 g. Mild centrifugation includes the conditions known for the skilled man in the art, said conditions allowing separation of the particles at the bottom of the recipient but preserving the functional structure of the same (i.e. not damaging or disrupting the particles and/or proteins conforming them).

The term "mechanically stable", which means that the cluster of self-contained proteins remains structured when submitted to particular sonication conditions. For "remain structured" is to be understood that the assembled self-contained proteins that form part of the cluster do not lose their tridimensional configuration; that resembling natural inclusion bodies.

The term "denatured form" or "denatured proteins" means that the proteins have lost the at least the quaternary structure and tertiary structure, which is present in their native state, by application of some external stress or compound such as a strong acid or base, a concentrated inorganic salt, an organic solvent (e.g., alcohol or chloroform), radiation or heat.

The term "functional protein" when used in this description, relates to the widely acceped meaning of a protein that maintains, works or provides its purposed activity. For example if a protein has the capability of fluorescence emission, the protein is functional if it can emit such fluorescence, which is the purpose for which it was used or designed. If on the other hand usual function of the protein involves inhibition of growth factors, the functional protein will maintain such property.

As used herein, "targeting molecule" refers to a molecule having specificity for a particular cell, tissue, or organ. Preferred examples of targeting molecules include but are not limited to antibodies, growth factors, and polysaccharides.

The particle is a nanoparticle or a microparticle. The term "nanoparticle" as used herein, refers to a particle with at least two dimensions at the nanoscale, particularly with all three dimensions at the nanoscale. For analogy, the term "microparticle" as used herein, refers to a particle with at least two dimensions at the microscale, particularly with all three dimensions at the microscale. In a particular embodiment, the particle is from 50 nm to 50 $\mu$m. In particular, from 60 nm to 10 $\mu$m. More in particular, from 60 nm to 5 $\mu$m. Even more in particular, from 60 nm to 1 $\mu$m, even more in particular from 60 nm to 900 nm. Other more particular sizes are from 60 nm to 200 nm. Protein particles of the invention and produced according to the methods disclosed therein include a distribution of microparticles and of nanopartiples. Thus, the term "protein nano- or microparticle" or the term protein-lipid "nano- or microparticle", relates to a mixture or distribution of particles of different sizes including nanoparticles, microparticles or combinations of nano and microparticles.

As regards the shape of the nanoparticles or microparticles described herein, there are included spheres, polyhedral and rod-shape. Particularly, when the nanoparticle or microparticle is substantially rod-shaped with a substantially circular cross-section, such as a nanowire or a nanotube, microwire or microtube, the "nanoparticle" or "microparticle" refers to a particle with at least two dimensions at the nanoscale or microscale, these two dimensions being the cross-section of the nanoparticle or the microparticle.

In a particular embodiment of the first aspect, optionally in combination with any of the embodiments provided above or below, the particle is spherical or pseudospherical.

As used herein, the term "size" refers to a characteristic physical dimension. For example, in the case of a nanoparticle/microparticle that is substantially spherical, the size of the nanoparticle/microparticle corresponds to the diameter of the nanoparticle/microparticle. When referring to a set of nanoparticles/microparticles as being of a particular size, it is contemplated that the set can have a distribution of sizes around the specified size. Thus, as used herein, a size of a set of nanoparticles/microparticles can refer to a mode of a distribution of sizes, such as a peak size of the distribution of sizes. In addition, when not perfectly spherical (pseudospherical), the diameter is the equivalent diameter of the spherical body including the object. This diameter is generally referred as the "hydrodynamic diameter", which measurements can be performed using a Wyatt Möbius coupled with an Atlas cell pressurization system. Transmission Electron Microscopy (TEM) images do also give information regarding diameters.

As used herein, the indefinite articles "a" and "an" are synonymous with "at least one" or "one or more." Unless indicated otherwise, definite articles used herein, such as "the" also include the plural of the noun.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The term "cosmetic effective amounts" is defined as any amount sufficient to significantly improving the cosmetic appearance of the skin without substantial irritation, but low enough to avoid serious side effects (at a reasonable benefit/risk ratio), within the scope of sound medical judgement. The safe and effective amount of the particles of the invention will vary with the age and physical condition of the consumer, the condition of the skin, the duration of the treatment, the nature of any concurrent treatment, the specific combination of active ingredients employed, the particular cosmetically acceptable carrier utilized, and like factors in the knowledge and expertise of any attending physician.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "cosmetically acceptable" or "dermatological acceptable" which is herein used interchangeably refers to that excipients or carriers suitable for use in contact with human skin without undue toxicity, incompatibility, instability, allergic response, among others.

As above indicated, present invention relates to protein nano- or microparticles comprising clusters of one or more types of assembled self-contained proteins, wherein the nano-or micro particles:

- have a size, measured as hydrodynamic diameter, from 50 nanometers (nm) to 50 micrometers ($\mu$m);
- are mechanically stable, which means that the cluster of self-contained proteins remains structured when submitted at sonication conditions including 5 rounds of 40 seconds; 0.5 of pulse on; 0.5 of pulse off and a wave width of 10 % in a high intensity sonicator Branson sonifier 450,with 3 mm-diameter titanium probe;
- are in the form of a precipitated pellet in aqueous media, when centrifuged at 15.000 g at a temperature from 4 °C to 30 °C; and
- they release an amount of assembled self-contained proteins lower than 50 % by weight in relation to the total weight of assembled self-contained protein, within 24 hours and when resuspended in an aqueous media at physiological temperature.

In some particular embodiments, the particles release at least 50% by weight of the assembled self-contained proteins within at least 15 days while resuspended in aqueous media at physiological temperature.

In a particular embodiment, protein nano-or microparticles comprise a cluster made of assembled self-contained proteins of one or more types, which proteins are so assembled or aggregated due to the presence of salts of divalent cations within the scaffold (or cluster). Thus, in a particular embodiment, the protein nano- and/or microparticle according to the first aspect further comprise one or more salts of divalent cations.

Salts of divalent cations include single and multiple salts (i.e. double salts), and combinations thereof. In a more particular embodiment the divalent cations of the salts are selected from the group consisting of $Be^{2+}$, $Mg^{2+}$, $Ca^{2+}$, $Sr^{2+}$, $Ba^{2+}$, $Ra^{2+}Zn^{2+}$, $Cu^{2+}$ and $Ni^{2+}$, and combinations thereof. In a particular embodiment, divalent cations of the salts are alkaline-earth cations. The salts are, in particular embodiment inorganic salts of divalent cations, more in particular of $Be^{2+}$, $Mg^{2+}$, $Ca^{2+}$, $Sr^{2+}$, $Ba^{2+}$, $Ra^{2+}$, $Zn^{2+}$, $Cu^{2+}$ and $Ni^{2+}$, and combinations thereof. Particular salts include $CaCl_2$, $ZnCl_2$,

NiCl$_2$, and combinations thereof.

In another particular embodiment of the first aspect, optionally in combination with any embodiment above or below, the assembled self-contained proteins comprise one or more amino acids that due to the presence of charge at physiological pH and/or of the presence of aromatic or heteroaromatic structures can coordinate with the divalent cations. In another particular embodiment of the first aspect, optionally in combination with any embodiment above or below, the assembled self-contained proteins comprise one or more histidine residues. Thus, they are histidine-containing proteins.

In another particular embodiment, optionally in combination with any embodiment above or below, the cluster of protein molecules comprises His-tagged proteins. His tagged proteins are also known as histidine-rich proteins, which are proteins, usually recombinant proteins, comprising a polyhistidine-tag. This polyhistidine-tag is an amino acid motif in proteins that consists of at least six histidine (His) residues, often at the N- or C-terminus of the protein. Nonetheless, some proteins also comprise these at least six histidine residues in the middle of their amino acid sequence, such as in loop regions. This motif of histidine residues is also known as hexa histidine-tag, 6xHis-tag, His6 tag and by the trademarked name His-tag (registered by EMD Biosciences). His-tagged proteins according to present invention also include natural proteins that comprise high amounts of histidine amino acid in their sequences.

In another particular embodiment of the first aspect, optionally in combination with any embodiment above or below, the assembled self-contained proteins comprises non-fibrous proteins or, in other words, the proteins configuring the cluster of assembled proteins are selected from the group consisting of membrane proteins, globular proteins, disordered proteins, and combinations thereof.

In another particular embodiment of the first aspect, the protein nano- or microparticle comprises self-contained proteins that are therapeutic proteins, said therapeutic proteins optionally covalently linked and/or conjugated to one or more additional different therapeutic agent. In this particular embodiment, the same proteins configuring the cluster or scaffold of the nano- or microparticle are the therapeutics that can be delivered when the particle is sustainly solubilized at physiological conditions within a predetermined period of time, which time is function not only of the nature of the protein itself but also of the other compounds that can be accompanying the self-contained proteins configuring the cluster, such as other functional proteins, small drug molecules or therapeutic agents, or accompanying lipid structures, as a mode of non-limiting examples.

Particular therapeutic proteins, which mean that they are peptides (4 to 30 amino acids) or polypeptides (from 31 amino acids) with a proved therapeutic effect, include enzymes, hormones (i.e. insulin, growth hormones, etc.), hematopoietic growth factors (erythropoietin and derivatives, cell stimulating factors, such as granulocyte colony stimulating factors, etc.), interferons (Interferons-α, -β, -γ), blood factors (i.e. Factor VIII, Factor IX), thrombolytic agents (tissue plasminogen activator), antibodies (monoclonal and polyclonal antibodies), tumor necrosis factors, virus surface antigens, and hormones (insulin, glucagon, gonadotrophins, growth hormone), cell membrane receptors and corresponding secreted versions (i.e. klotho protein). Therapeutic proteins also encompass peptide aptamers and affimer molecules.

Thus, particles of the invention can be used as medicaments, in particular in diseases where the effective amounts of the therapeutic proteins are the active ingredients.

In a particular embodiment, the self-contained proteins are therapeutic proteins with a therapeutic effect selected from an anti-tumor effect, an anti-inflammatory effect, an antibiotic effect, an anti-fungal effect, an anti-viral effect, a growth factor effect, a cell growth inhibitor effect, an anti-platelet effect, an anti-thrombotic effect, a thrombolytic effect, and combinations thereof. In a more particular embodiment, the self-contained proteins are therapeutic proteins with an anti-tumor effect.

In yet another particular embodiment, optionally in combination with the embodiments above or below, the protein nano- or microparticle is a protein-lipid nano- or microparticle that comprises a cluster of assembled self-contained proteins and one or more types of lipids assembled with the self-contained proteins. In this particular embodiment the nano-or microparticle comprises a cluster comprising proteins and lipids that configure a tridimensional scaffold (i.e structure) with self-contained one or more proteins and with lipids associated to these proteins. Interacting with this cluster comprising lipids, other compounds are present in another particular embodiment, said compounds selected from functional proteins different from that of the self-contained ones and/or small drugs (therapeutic agents). These other compounds are disposed within interstitial spaces of the cluster of lipids and proteins and/or are adhered (adsorbed) to the cluster, by means of ionic interactions, van der Waals forces, or they are covalently linked.

Thus, in another more particular embodiment of the protein-lipid nano- or microparticle:

- the self-contained proteins are denatured proteins and together with the assembled lipids configure a tridimensional scaffold; and
- the particle further comprises one or more functional proteins disposed within the tridimensional scaffold and/or adhered (adsorbed) thereto.

In another particular embodiment of the protein-lipid nano- or microparticle the one or more functional proteins are therapeutic proteins, optionally covalently linked and/or conjugated to a one or more additional different therapeutic agent.

In a more particular embodiment, the one or more lipids are selected from the group consisting of fatty acids, glycerophospholipids, sterols, sphingolipids, and combinations thereof. In a more particular embodiment, the glycerophospholipids are selected from phosphatidylcoline, phosphatidylserine, phosphatidylethanolamine, phosphatidylinositol, and combinations thereof. The sterols are in particular selected from cholesterol, phytosterol, and combinations thereof. The sphingolipids are selected from sphingosine, ceramide, sphingomyelin, glucosyl cerebroside, and combinations thereof. In particular the lipids are those encompassed in the three known categories; phospholipids, glycolipids and cholesterol. The one or more lipids are, in a particular embodiment, the common lipids in the membranes of cells of living beings.

In another particular embodiment of the first aspect, the one or more functional proteins are therapeutic proteins, which mean that they are peptides (4 to 30 amino acids) or polypeptides (from 31 amino acids) with a proved therapeutic effect, as previously disclosed for the self-contained proteins. Examples of therapeutic proteins include, again, enzymes, hormones (i.e. insulin, growth hormones, etc.), hematopoietic growth factors (erythropoietin and derivatives, cell stimulating factors, such as granulocyte colony stimulating factors, etc.), interferons (Interferons-$\alpha$, -$\beta$, -$\gamma$), blood factors (i.e. Factor VIII, Factor IX), thrombolytic agents (tissue plasminogen activator), antibodies (monoclonal and polyclonal antibodies), tumor necrosis factors, virus surface antigens, and hormones (insulin, glucagon, gonadotrophins, growth hormone), cell membrane receptors and corresponding secreted versions (i.e. klotho protein). Therapeutic proteins also encompass peptide aptamers and affimer molecules.

In another particular embodiment, the functional proteins are configured as nanoparticles of self-assembled proteins in the presence of divalent cations, more in particular histidine-containing proteins, in the presence of divalent cations selected from the group consisting of $Be^{2+}$, $Mg^{2+}$, $Ca^{2+}$, $Sr^{2+}$, $Ba^{2+}$, $Ra^{2+}Zn^{2+}$, $Cu^{2+}$ and $Ni^{2+}$, and combinations thereof. This particular embodiment will be in protein microparticles, in particular, with a size from 1 $\mu$m to 50 $\mu$m.

In another particular embodiment of the first aspect, the one or more functional proteins disposed within the scaffold or cluster of self-assembled proteins or of proteins and lipids can additionally be covalently linked to this scaffold. In this particular case and when the functional protein is the active to be released, the covalent links are hydrolysable bonds. More in particular, they are hydrolysable at physiological conditions (i.e., ester bonds, amide bonds).

In another particular embodiment, these one or more functional proteins are proteins covalently linked and/or conjugated to a therapeutic agent, or to more than one therapeutic agent. This means that besides the functional protein has a therapeutic effect *per se,* other molecules with therapeutic effect are also combined (covalently linked or not with the functional protein). These therapeutic agents are, in a particular embodiment small molecules ($\leq$ 900 Da). In the alternative, the therapeutic agent linked or conjugated to the functional protein is another protein or peptide. In yet another particular embodiment the therapeutic agent and the functional protein constitute a fusion protein and comprise two or more polypeptide fragments operatively linked and produced by recombinant technologies (i.e exogenous expression in expressing cells). In this later case when fusion proteins are used one or more of the fused polypeptides are functional proteins and have therapeutic effect. The therapeutic effect of the fusion protein is in a particular embodiment a polyvalent therapeutic effect, which means that each of the fused polypeptides exerts a different effect that can be complementary to each other. In another particular embodiment, all the fused proteins exert the same therapeutic effect (i.e. all fused parts have an anti-tumor effect).

In yet another particular embodiment, the therapeutic agent, embedded or adhered to the cluster of the nano-or- microparticle, linked and/or conjugated to the one or more functional proteins or to the self-contained proteins in the particles, are selected from the group consisting of an anti-tumor agent, an anti-inflammatory molecule, an antibiotic, an anti-fungal molecule, an anti-viral molecule, a growth factor, a cell growth inhibitor, an anti-platelet agent, an anti-thrombotic agent, a thrombolytic agent, and combinations thereof.

Non-limiting examples of therapeutic agents of different nature, embedded, adhered or linked in the microparticles or nanoparticles are listed below:
Non-limiting examples of antibiotics include $\beta$-lactam antibiotics (penicillin derivatives, monobactams, and carbapenems ; polymyxins, such as colistin; rifamycins; lipiarmycins; quinolones; sulfonamides; macrolides; lincosamides; tetracylines; bactericidal aminoglycosides; cyclic lipopeptides, such as daptomycin; glycylcylines, such as tigecycline; oxazolidinones, such as linezolid; and lipiarmycins, such as fidaxomicin.

In a similar way, non-limiting examples of anti-fungal molecules, are amphotericin B, candicidin, filipin, hamycin, natamycin, nystatin and rimocidin; azole anti-fungals, such as imidazoles, e.g. bifonazole, butoconazole, clotrimazole, econazole, fenticonazole, isoconazole, miconazole, omoconazole, oxiconazole, sertaconazole, sulconazole and tioconazole; triazoles, e.g. albaconazole, fluconazole, isavuconazole, itraconazole, posaconazole, ravuconazole, terconazole and voriconazole; and thiazoles, e.g. abafungin; allylamines, such as amorolfin, butenafine, naftifine and terbinafine; echinocandins, such as anidulafungin, caspofungin and micafungin; benzoic acid; ciclopirox olamine; flucytosine; griseofulvin; tolnaftate and undecylenic acid.

Non-limiting examples of anti-viral molecules, include virus-assisted protein (VAP) anti-idiotypic antibodies;

amantadine; rimantadine; pleconaril; acyclovir; zidovudine (AZT); lamivudine; integrase; fomivirsen; rifampicin; zanamivir and oseltamivir, and anti-parasitic molecules, such as mebendazole; pyrantel pamoate; thiabendazole; diethylcarbamazine; ivermectrin; niclosamide; praziquantel; albendazole; praziquantel; rifampin; amphotericin B; melarosprol; elfornithine; metronidazole; tinidazole and miltefosin.

A further example of therapeutic agent include growth factors, such as adenomedullin (AM), angiopoietin (Ang), autocrine motility factor, bone morphogenetic proteins (BMPs), brain-derived neutrophic factor (BDNF), epidermal growth factor (EGF), erythropoietin (EPO), fibroblast growth factor (FGF), glial cell line-derived neutrophic factor (GDNF), granulocyte colony-stimulating factor (G-CSF), granulocyte macrophage colony-stimulating factor (GM-CSF), growth differentiation factor-9 (GDF9), hepatocyte growth factor (HGF), hepatoma-derived growth factor (HDGF), insulin-like growth factor (IGF), mystatin (GDF-8), nerve growth factor (NGF), platelet-derived growth factor (PDGF), thrombopoietin (TPO), transforming growth factor alpha (TGF-$\alpha$), transforming growth factor beta (TGF-$\beta$), tumor necrosis factor alpha (TNF-$\alpha$), vascular endothelial growth factor (VEGF), and placental growth factor (PIGF)

In yet a more particular embodiment, the anti-tumor agent is selected from one or more of the categorical group consisting of a chemotherapy agent, a cytotoxic agent (i.e either of polypeptide nature or a small drug), and antiangiogenic agent, a pro-apoptotic agent, an anti-metastatic agent or anti-proliferative agent (i.e an anti-mitotic agent), an immune stimulating agent (i.e promoting immune response towards tumors), a polypeptide encode by a tumor suppressor gene, a toxin, and combinations thereof.

Particular examples of anti-tumor agents, which are compounds that finally aim the fight of a cancer process in a patient include, but are not limited to, farnesyl transferase inhibitors; alkylating agents, such as nitrogen mustards, e.g. mechlorethamine, cyclophosphamide, melphalan, chlorambucil, ifosfamide and busulfan; nitrosoureas, e.g. N-nitroso-N-methylurea (MNU), carmustine (BCNU), lomustine (CCNU), semustine (MeCCNU), fotemustine and streptozotocin; tetrazines, e.g. dacarbazine, mitozolomide and temozolomide and aziridines, e.g. thiotepa, mytomycin, diaziquone (AZQ); and cisplatines, e.g. cisplatine, carboplatin and oxaplatin; antimetabolites, such as anti-folates, e.g. methotrexate and pemetrexed; fluropyrimidines, e.g. fluorouracil and capecitabine; deocynucleoside analogues, such as cytarabine, gemcitabine, decitabine, Vidaza, fludarabine, nelarabine, cladribine, clofarabine and pentostatine; and thiopurines, e.g. thiguanine and mercaptopurine; anti-microtubule agents, such as vinca alkaloids, e.g. vincristine, vinblastine, vinorelbine, vindesine and vinflunine; and taxanes, e.g. paclitaxel and docetaxel; and podophyllotxin; topoisomerase inhibitors, such as irinotecan, topotecan, captothecin, etoposide, doxorubicin, mitoxantrone, teniposide, novobiocine, merbarone and aclarubicin; cytotoxic antibiotics, such as antracyclines, e.g. doxorubicin, daumorubicin, epirubicin, idarubicin, pirarubicin, aclarubicin, mitoxantrone, actinomycin, bleomycin, plicamycin, and mitomycin. Other particular anti-tumor agents include antibodies, in particular monoclonal antibodies, such as trastuzumab, rituximab, alemtuzumab, Ibritumomab tiuxetan, bevacizumab, cetuximab, or panatimumab.

Other therapeutic agents include nucleic acid aptamers, peptide aptamers and affimers.

In yet another particular embodiment of the first aspect of the invention, the protein nano-or microparticle further comprises a targeting molecule linked to the cluster of assembled self-contained proteins. This targeting molecule has specificity for a particular cell, tissue, or organ to which the particles of the invention have to be delivered. Preferred examples of targeting molecules include but are not limited to antibodies, growth factors, and polysaccharides.

Present invention also encompasses a method for the synthesis of a protein nano- or microparticles as defined above, wherein the method comprises the following steps:

(a) mixing in a recipient one or more types of proteins in a polar solvent, more in particular a polar buffered solvent;
(b) submitting the mixture of step (a) to protein assembly conditions to obtain a protein-nano- or microparticle comprising a cluster of assembled self-contained proteins; and
(c) isolating the nano- or microparticle.

Thus, the invention encompasses a protein nano- or microparticle comprising a cluster of one or more types of assembled self-contained proteins, wherein the nano-or micro particle:

- has a size, measured as hydrodynamic diameter, from 50 nanometers (nm) to 50 micrometers ($\mu$m);
- is mechanically stable, which means that the cluster of self-contained proteins remains structured when submitted at sonication conditions including 5 rounds of 40 seconds; 0.5 of pulse on; 0.5 of pulse off and a wave width of 10 % in a high intensity sonicator Branson sonifier 450, with 3 mm-diameter titanium probe;
- is in the form of a precipitated pellet in aqueous media, when centrifuged at 15.000 g at a temperature from 4 °C to 30 °C; and
- it releases an amount of assembled self-contained proteins lower than 50 % by weight in relation to the total weight of assembled self-contained proteins within 24 hours and when resuspended in an aqueous media at physiological temperature;

and wherein the protein nano- or microparticle is obtainable by a method comprising the steps of:

(a) mixing in a recipient one or more types of proteins in a polar solvent, more in particular a polar buffered solvent;
(b) submitting the mixture of step (a) to protein assembly conditions to obtain a protein-nano- or microparticle comprising a cluster of assembled self-contained proteins; and
(c) isolating the nano- or microparticle.

In a more particular embodiment of the method of the second aspect, in step (b) the protein assembly conditions comprise the addition of salts to the mixture of step (a) at a final salt concentration of salts in the mixture allowing precipitation of the one or more proteins, and/or applying a protein-denaturation temperature.

In another more particular embodiment of the method of the second aspect, it comprises the following steps:

(a) mixing in a recipient one or more types of proteins in a polar solvent;
(b) adding to the mixture of step (a) a solution of salts of divalent cations at a final salt concentration of salts in the mixture allowing precipitation of the one or more proteins;
(c) isolating the precipitated one or more proteins from the solvent in which they were precipitated, which are protein nano- or microparticles comprising a cluster of assembled self-contained proteins, and optionally resuspending them in a fresh buffered solvent.

Fresh buffer means new polar solvent, comprising a buffer system for the pH control, and the salt concentration to have the ionic force (isotonic) required by the proteins.

Thus, in a particular embodiment of the protein nano- or microparticle of the first aspect, said protein nano- or microparticle is obtainable by a method comprising the steps of:

(a) mixing in a recipient one or more types of proteins in a polar solvent;
(b) adding to the mixture of step (a) a solution of salts of divalent cations at a final salt concentration of salts in the mixture allowing precipitation of the one or more proteins;
(c) isolating the precipitated one or more proteins from the solvent in which they were precipitated, which are protein nano- or microparticles comprising a cluster of assembled self-contained proteins, and optionally resuspending them in a fresh buffered solvent.

In a particular embodiment, step (c) of isolating is carried out by means selected from the group consisting of centrifugation, filtering, drying, and combinations thereof, the skilled man will know. In another more particular embodiment isolation is performed by beans of centrifugation.

In another particular embodiment of the method of the second aspect when salts of divalent cations are used the final ratio of moles of salt of divalent cation:moles of protein is comprised from 4:1 to 1000:1. This proportion will depend on the protein that is going to be used, and the skilled man will know how to accommodate the amounts. For instance, the number of histidine residues or of other amino acid residues that can made complex linkages with the divalent cations will be a variable when adjusting the proportions, mainly requiring low amounts of divalent cations when the number of this complexing to the cation residues is higher in the protein molecule. Thus, it will vary depending on the amino acids in the protein molecule that can chelate (coordinated linkage) with the divalent cations. In a particular embodiment of the method of the second aspect when salts of divalent cations are used, the one or more proteins comprise one or more histidine residues.

In another more particular embodiment, the final ratio of moles of salt of divalent cation:moles of protein is comprised from 5:1 to 500:1. More in particular from 10:1 to 500:1, even more in particular is from 20:1 to 200:1. Particular preferred ratios are selected from 5:1, 10:1, 20:1, 30:1, 40:1, 50:1, 60:1, 70:1, 100:1 and 150:1.

Particular salts of divalent cations are the same as indicated for the first aspect of the invention.

In another particular embodiment of the method, said method is for the synthesis of a protein-lipid nano- or microparticle as defined above and comprises the steps of:

(a) mixing in a recipient one or more types of proteins in a polar solvent;
(b) adding to the mixture of step (a) a solution of salts at a protein-denaturation salt concentration and/or submitting the mixture to protein-denaturation temperature allowing precipitation of the one or more proteins in denatured form (denatured proteins);
(c) isolating the precipitated one or more proteins from the solvent in which they were precipitated, and resuspending them in a fresh buffered solvent, said step of resuspending with a fresh solvent comprising:

(c.1) Mixing the precipitated protein with one or more lipids dissolved in an organic solvent and wherein the ratio of the amount of protein and lipids is from 0.8:1 to 1:0.8, more in particular 1:1;

(c.2) removing the organic solvent to obtain a dry film of denatured proteins and lipids on the surface of the recipient;

(c.3) suspending the dry film of step (c.2) with a buffered composition, said buffered composition optionally comprising one or more functional proteins in an aqueous media, while agitating the mixture under a controlled temperature from 4°C to 8 °C, to obtain a protein-lipid nano- or microparticle comprising a cluster of assembled self-contained proteins and one or more types of lipids assembled with the self-contained proteins, said cluster optionally comprising one or more functional proteins embedded and/or adsorbed within the cluster of assembled self-contained proteins and lipids;

(c.4) separate the protein-lipid nano- or microparticle from the remaining buffered composition.

Thus, in a particular embodiment of the protein nano- or microparticle of the first aspect, said protein-lipid nano- or microparticle is obtainable by a method comprising the steps of:

(a) mixing in a recipient one or more types of proteins in a polar solvent;

(b) adding to the mixture of step (a) a solution of salts at a protein-denaturation salt concentration while and/or submitting the mixture to protein-denaturation temperature allowing precipitation of the one or more proteins in denatured form (denatured proteins);

(c) isolating the precipitated one or more proteins from the solvent in which they were precipitated, and resuspending them in a fresh buffered solvent, said step of resuspending with a fresh solvent comprising:

(c.1) Mixing the precipitated protein with one or more lipids dissolved in an organic solvent and wherein the ratio of the amount of protein and lipids is from 0.8:1 to 1:0.8, more in particular 1:1;

(c.2) removing the organic solvent to obtain a dry film of denatured proteins and lipids on the surface of the recipient;

(c.3) suspending the dry film of step (c.2) with a buffered composition, said buffered composition optionally comprising one or more functional proteins in an aqueous media, while agitating the mixture under a controlled temperature from 4°C to 8 °C, to obtain a protein-lipid nano- or microparticle comprising a cluster of assembled self-contained proteins and one or more types of lipids assembled with the self-contained proteins, said cluster optionally comprising one or more functional proteins embedded and/or adsorbed within the cluster of assembled self-contained proteins and lipids;

(c.4) separate the protein-lipid nano- or microparticle from the remaining buffered composition.

In a particular embodiment of the methods, the polar solvent of step (a) is an aqueous buffered composition, more in particular an aqueous buffered composition at a pH from 6.8 to 7.5. More in particular is water with a buffer to adjust pH. Particular buffers include phosphate-buffered saline (containing disodium hydrogen phosphate, sodium chloride and, in some formulations, potassium chloride and potassium dihydrogen phosphate), Tris-glycine or Tris-HCl. Aqueous buffered compositions area also termed in this description as an "aqueous media".

In a particular embodiment of the methods in which protein-denaturation temperature is applied allowing precipitation of the proteins, said temperature is comprised from 60 °C to 120 °C. These temperatures allow obtaining denatured proteins. In another particular embodiment of the method, the protein-denaturation salt concentration is at least of 0.5 M in the final mixture of step (b). More in particular yet, step (b) of the method for the synthesis of protein-lipid nano- or microparticles is carried out at a temperature from 90 °C to 120 °C and at a salt concentration is at least of 0.5 M.

Particular salts in the method for synthesis of a protein-lipid nano- or microparticle, are selected from salts of monovalent cations, salts of divalent cations and combinations thereof. Particular salts of divalent cations are selected from the group previously indicated for the first aspect. More in particular salts are selected from the group consisting of NaCl, KCl, LiCl, $MgCl_2$, $CaCl_2$, and combinations thereof.

In another particular embodiment of the method for the synthesis of a protein-lipid nano-or microparticle, in step (c) of isolation and resuspension of the precipitated one or more proteins, step (c1) is performed with an organic solvent selected from the group consisting of ethanol, mixtures of chloroform and methanol, hexane, chloroform, methanol, and combinations thereof.

In another particular embodiment of the method for the synthesis of a protein-lipid nano-or microparticle, in step (c) of isolation and resuspension of the precipitated one or more proteins, step (c3) is performed with an aqueous buffered composition, more in particular an aqueous buffered composition at a pH from 6.8 to 7.5. This buffered composition comprises, in another more particular embodiment, one or more functional proteins in the aqueous media, in such a way that when the film of denatured proteins and lipids is resuspended with said buffer, the clusters are formed and they comprise the additional functional proteins embedded in the cluster or adhered (adsorbed) thereto

Particular recipients to carry out the methods are of a material selected from the group consisting of glass, polypropylene (HDPP or LDPP), and polyethylene. In another particular embodiment, the support is glass. Glass is preferably used when particular organic solvents that could dissolve some plastic types (polymer) are used.

The invention also relates to dry films comprising one or more lipids and one or more denatured proteins, which are self-assembled and configure a tridimensional cluster, net, said film disposed on a support.

For dry films is to be understood that the percentage of humidity in the film is from 0 % to 30% of relative humidity, measured according to known standard methodologies.

In another particular embodiment of the film, the support is a flat surface of a material selected from the group consisting of glass, polypropylene (HDPP or LDPP), and polyethylene. In another particular embodiment, the support is glass. As indicated, glass is preferably used when particular organic solvents that could dissolve some plastic types are used.

The dry film comprising one or more lipids and one or more denatured proteins, which are self-assembled and configure a tridimensional cluster is obtainable by a method comprising:

(a) mixing in a recipient one or more types of proteins in a polar solvent;
(b) adding to the mixture of step (a) a solution of salts at a protein-denaturation salt concentration and/or submitting the mixture to protein-denaturation temperature allowing precipitation of the one or more proteins in denatured form (denatured proteins);
(c) isolating the precipitated one or more proteins from the solvent in which they were precipitated, and mixing the precipitated protein with one or more lipids dissolved in an organic solvent and wherein the ratio of the amount of protein and lipids is from 0.8:1 to 1:0.8, more in particular 1:1; and
(d) removing the organic solvent to obtain a dry film of denatured proteins and lipids on the surface of the recipient.

This film of the invention can be further resuspended with a buffered composition optionally comprising one or more desired functional proteins, as indicated above, to obtain the protein-lipid nano- or micro particles comprising the scaffold (or clusters) of assembled self-contained proteins and one or more types of lipids assembled with the self-contained proteins, said cluster optionally comprising one or more functional proteins embedded and/or adsorbed within the cluster of assembled self-contained proteins and lipids. Thus, resuspended film allow obtaining particles comprising the cluster with one or more lipids and one or more denatured proteins, which are self-assembled and configure a tridimensional net; and the one or more functional proteins disposed within the scaffold or adhered thereto.

Particular organic solvents are as disclosed above for the method of obtaining the nano-or microparticles.

The protein nano- or microparticle of the invention is for use as a medicament as an additional aspect.

In a particular embodiment, the particles are for use in the treatment of a disease selected from the group consisting of cancer, an immune disease, neurodegenerative disease, and combinations thereof. This embodiment can also be formulated as the use of the protein nano- or microparticle as defined above for the manufacture of a medicament for the treatment or prevention of a disease selected from the group consisting of cancer, an immune disease, neurodegenerative, and combinations thereof. The present invention also relates to a method for the treatment or prevention of a disease selected from the group consisting of cancer, an immune disease, neurodegenerative, and combinations thereof, comprising administering a pharmaceutically effective amount of the protein nano-or microparticle as defined above, together with pharmaceutically acceptable excipients or carriers, in a subject in need thereof, including a human. Particular cancers include colorectal cancer.

As indicated, one aspect of the invention is a pharmaceutical composition comprising a therapeutically effective amount of the protein nano- or microparticle disclosed above together with pharmaceutically acceptable excipients or carriers.

In particular embodiments, the pharmaceutical composition of the invention can be formulated in several forms that include, but are not limited to, solutions, tablets, capsules, granules, suspensions, dispersions, creams, ointments, powders, lozenge, chewable candy, candy bar, concentrate, drops, elixir, emulsion, film, gel, granule, chewing gum, jelly, oil, paste, pastille, pellet, soap, sponge, suppository, syrup, chewable gelatin form, or chewable tablet.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the protein nano- or microparticle, the pharmaceutically acceptable excipients, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, and flavoring agents can be present in the composition, according to the judgment of the formulator.

The invention also relates to cosmetic compositions comprising a cosmetic effective amount of the protein nano- or microparticles disclosed above together with one or more appropriate cosmetically acceptable excipients or carriers. Particular cosmetic compositions comprise protein nano- or microparticles comprising clusters of assembled self-contained proteins and optionally other functional proteins embedded or adhered to said cluster, one or more of any of these proteins, either the assembled self-contained or the functional embedded or adhered, being selected from proteins commonly used in cosmetics and selected from collagen of any type, growth factors, elastin, fibrin, among others. These proteins and so the cosmetic compositions comprising them can be used in particular for skin care, in particular for ameliorating at least one of the following symptoms: roughness, flakiness, dehydration, tightness, chapping, and lack of elasticity.

Other more particular cosmetic compositions comprise additional cosmetic active ingredients adhered or embedded in the nano- or microparticles. Non-limiting examples of cosmetic actives include plant extracts, plant cell lysates, anti-wrinkle compounds, hydrating compounds, whitening compounds, cicatrisation compounds, anti-cellulite compounds, skin-tightening compounds, antioxidant compounds, etc. These entire compounds can be of different nature, even of peptide nature or isolated amino acids. They can also be derived from plant compounds, such as phytosterols, polyphenols, terpenes, etc., or of lipid nature, such as fatty acids, or even they can derive from nucleic acids. Also compounds of polysaccharide structure can be used (i.e. hyaluronic acid compounds), or carboxylic acids. Therefore, all those actives providing a cosmetic effect can be used in the cosmetic compositions of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

**Examples**

Example 1. Method for preparing protein microparticles and nanoparticles (synthetic IBs), using as pattern the fusion protein T22-GFP-H6.

All process is to be carried out under sterility conditions.

Materials:

Sterile double distilled water. Sterile NaCl solution. Filtered PBS buffer 1x and concentrated and sterile solution of protein nanoparticles (NPs). Solution of lipids (egg phosphatidyl choline (Sigma Aldrich) or lipids found in natural cell membranes) in chloroform: methanol (2: 1) or organic solvents that homogeneously dilute the lipids used

1-Two solutions (solution A and B) of equal concentration of fusion protein T22-GFP-H6 (SEQ ID NO: 1) in double distilled water were prepared. (A concentration of 1mg / mL already used for in vitro tests is suggested). Solution A contained NaCl (0.5-2 M) and solution B did not contain salt.
SEQ ID NO: 1 corresponds to the following sequence, from N to C-terminal:

M R R W C Y R K C Y K G Y C Y R K C R G G S S R S S S K G E E L F T G V V P I L V E L D G

D V N G H K F S V S G E G E G D A T Y G K L T L K F I C T T G K L P V P W P T L V T T L T Y

G V Q C F S R Y P D H M K R H D F F K S A M P E G Y V Q E R T I S F K D D G N Y K T R A E

V K F E G D T L V N R I E L K G I D F K E D G N I L G H K L E Y N Y N S H N V Y I T A D K Q K

N G I K A N F K I R H N I E D G S V Q L A D H Y Q Q N T P I G D G P V L L P D N H Y L S T Q S

A L S K D P N E K R D H M V L L E F V T A A G I T H G M D E L Y K H H H H H H

T22-GFP-H6 was in form of nanoparticles (12 nm) formed through assembly of GFP-containing monomers produced in E. coli in the presence of divalent cations, as disclosed by Lopez-Laguna et al., "Assembly of histidine-rich protein materials controlled through divalent cations", Acta Biomaterialia 2019, vol. no. 83, pp.:257-264.

2-Solution A was heated for 20 to 40 min at 100 ° C.

3-Subsequently it was centrifuged at 15.000g for 30 min at 4°C. The presence of pellets corroborated the precipitation of the protein. The well-labeled supernatant was separated and stored to evaluate the presence of non-precipitated protein residues. Subsequently, two more centrifugations were carried out in the same conditions as before (15.000g for 30 min at 4°C) and resuspending both times in the same initial volume of double distilled water.

4-Final pellet was separated from the supernatant (with a metal spatula) and placed in the bottom of a glass container (*). Subsequently, lipid (egg phosphatidylcholine (Sigma Aldrich)) was added. The volume of lipid contained the same amount (in milligrams) of protein in the glass tube. In addition, a volume of lipid as small as possible is recommended for this step. So that, the lipid in the organic solvent was highly concentrated (10 mg of lipid / ml resuspended in chloroform: methane 2.1)
(*) The need to operate in this stage in a glass container was due to the plastic dissolving in chloroform / methanol.

5- Since the mixture of lipid and protein contained chloroform / methanol, these solvents were removed by means of N2 flow. The mixture was dried until a film of protein and lipid was completely dried (residual humidity from 0 % to 30 %) at the bottom of the glass tube. This film (scaffold of self-assembled protein and lipid molecules that configures a porous tridimensional net) can be stored if desired to be used in the future.

6-Later this film of protein and lipid was resuspended with a volume of the functional (which means that it works or provides the purposed activity) fluorescent protein T22-GFP-H6 of SEQ ID NO: 1 (1 mg / ml of buffered solution) in non-denatured form. The sample was mixed to a visible homogeneity and then the entire volume was transferred back to a plastic container to continue the procedure.

7-The sample was kept at 2 to 8°C (in cold chamber) throughout the night, to allow the interaction of the functional protein with the proteolipid film.

8-The next day the sample was centrifuged to 15000g for 30 min at 4 ° C to release the functional protein not bound. This centrifugation was repeated twice more. The supernatant was saved to evaluate how much protein did not bind to this film.

9-The last pellet contained the protein-lipid nano/microparticles according to the invention. This sample can be stored at -80°C if not used.

Therefore, preparation of synthetic IBs was performed by a method comprising the following steps:

(a) mixing one or more denatured proteins with one or more lipids previously dissolved in an organic solvent until an homogeneous mixture is obtained, wherein the ratio of the amount of protein and lipids is 1:1;
(b) removing the organic solvent to obtain a dry film of denatured proteins and lipids
(c) suspending the dry film of step (b) with composition comprising one or more functional proteins in an aqueous media while agitating the mixture under a controlled temperature from 4°C to 8 °C to obtain the protein-lipid nano- or microprotein comprising the scaffold (or cluster) and the one or more functional proteins disposed within, embedded or adhered thereto in the surface.
(d) separate the protein-lipid nano- or microprotein from the remaining aqueous media.

With this method a scaffold or cluster of self-assembled protein molecules configuring a tridimensional net was obtained, said scaffold comprising also lipids as in the natural IBs.; One functional protein (i.e. T22-GFP-H6) was added within a buffered solution and it was let enough time to allow it to be embedded or adsorbed (adhered) in the tridimensional net.

Example 2. Characterization of protein microparticles and nanoparticles (synthetic IBs)

Size and morphology:

Protein-lipid particles of Example 1 were analyzed by dynamic light scattering (DLS) and Field Emission Scanning Electron Microscopy (FESEM).

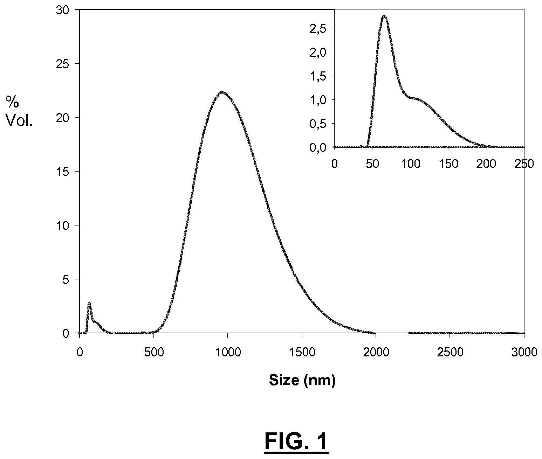

As can be seen in FIG. 1, which is a graphic of detected diameter (calculated by DLS) and the relative abundance of these diameters (% volume),the most of protein-lipid particles had a size around 1000 nm and they included particles from around 66 nm to 115 nm (small chart in FIG. 1).

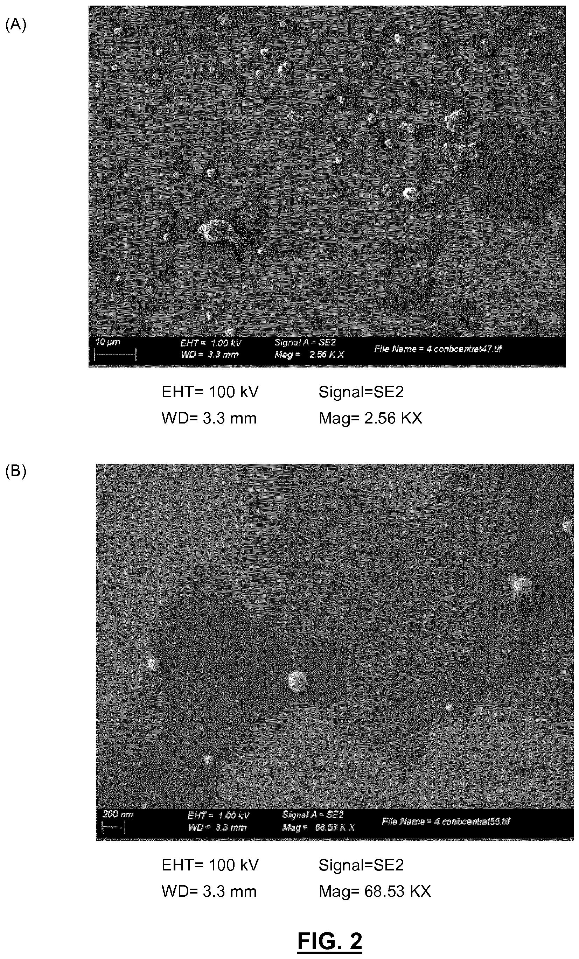

FIG. 2 shows FESEM images corroborating data of FIG. 1. FIG. 2 (A) shows synthetic IBs of big size and even some nanoparticles not previously detected. FIG. 2 (B) illustrates the nanoparticles of lower size also detected by DLS technology (FIG. 1). Images were obtained with the following adjusted device parameters: Electron high tension, (EHT) = 1.00 kV; working distance (WD) = 3.3 mm; Signal A = secondary electron (SE2); Mag = 2.56 K X.

Thus, the particles of the invention have a size from 60 nm to 2000 nm, within the range of bacterial inclusion bodies.

Example **3**. Fluorescence detection of T22-GFP-H6 in protein-lipid microparticles and nanoparticles of the invention

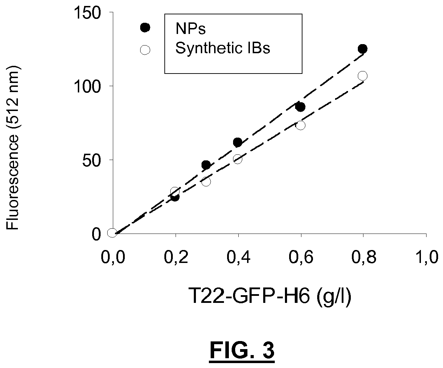

Fluorescence was evaluated with Cary Eclipse spectrofluorimeter (Agilent Technologies, Mulgrave, Australia), at a wave length of 512 nm] Data are depicted in FIG. 3, wherein for different concentrations of T22-GFP-H6 (g/l) relative intensity of fluorescence emitted at 512 nm is depicted for protein-lipid microparticles and nanoparticles of Example 1 (synthetic IBs; light circles). As control, fluorescence of the fusion protein in free form (not embedded in porous of lipid-protein scaffold) was also determined (NPs; dark circles).This free fusion protein was in form of nanoparticles (12 nm) formed through assembly of GFP-containing monomers produced in E. coli in the presence of divalent cations, as disclosed by López-Laguna et al., "Assembly of histidine-rich protein materials controlled through divalent cations", Acta Biomaterialia 2019, vol. no. 83, pp.:257-264.

Fluorescence was slightly lower in the particles in relation with the fluorescence of the protein nanoparticles originating them.

Example 4. Protein release from protein-lipid microparticles and nanoparticles of the invention (synthetic IBs)

For the analysis of soluble protein delivery of the particles obtained using the method of Example 1, artificial IBs in pellet format were resuspended in their corresponding buffers (without any additional protein) at a final concentration of 0.1 mg/mL and incubated without agitation at 37°C (physiological temperature). Aliquots of 150 $\mu$l were taken at defined times (from 0 to 10 days) and centrifuged for 15 min, 4°C at 15000g. Soluble and insoluble fraction were separated and further processed by Tris-Glycine eXtended (TGX) Stain-Free electrophoretic gels and Western Blot. In all cases, isolated insoluble fractions were then resuspended in their respective buffer at the same final volume than their soluble counterpart for comparison.

Release of T22-GFP-H6 (SEQ ID NO: 1) in the cluster of protein-lipid microparticles and nanoparticles of Example 1 was determined.

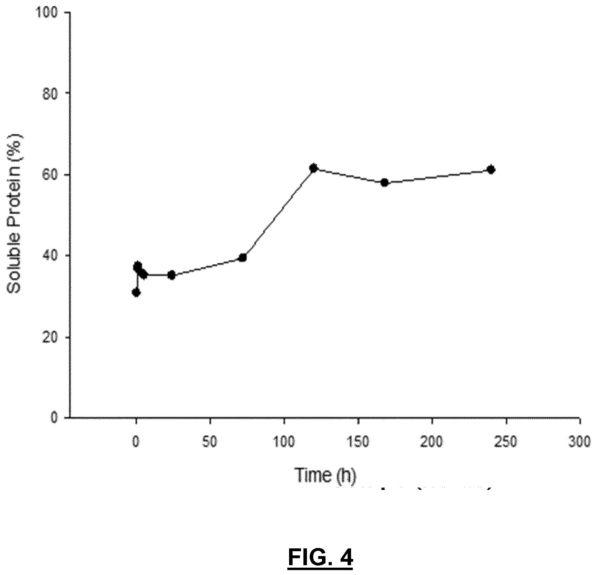

FIG. 4 shows the amount of released protein in % (i.e. release of functional fluorescent T22-GFP-H6) along time (Time in hours (h)).

A percentage by weight of 40% of protein in relation to the total protein was in the soluble fraction; meanwhile 60% remain in the form of a pellet.

Example 5. Internalization of protein-lipid microparticles and nanoparticles of the invention (synthetic IBs) into tumor cells

The capability of IBs to penetrate tumor cells expressing was measured with in HeLa cell line (ATCC® CCL-2™, commercially available) expressing the cytokine receptor CXCR4 (CXCR4+ cells).

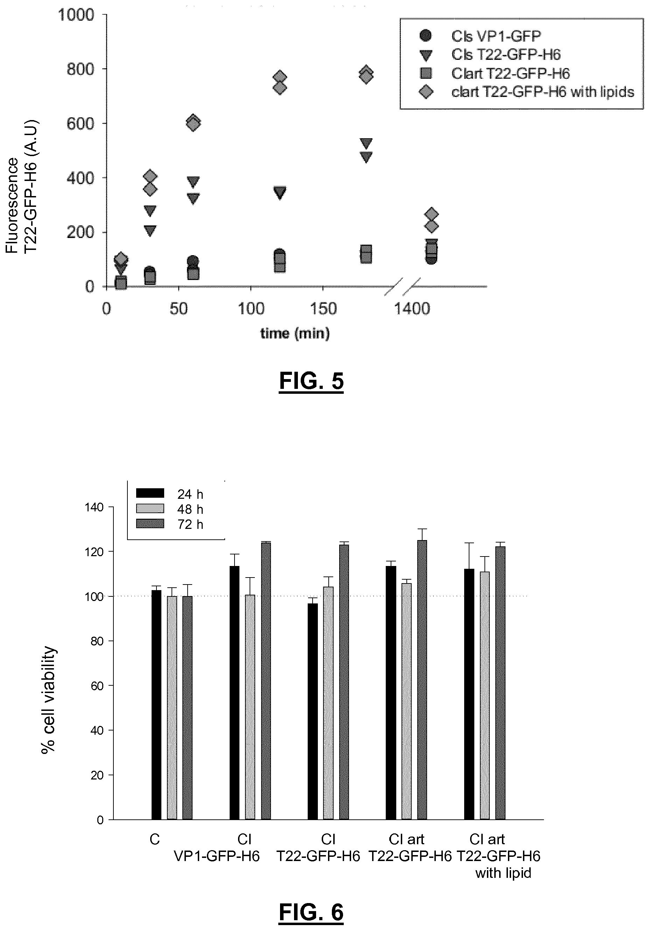

FIG. 5 is a graph with the detected fluorescence into the cells, determined by spectrofluorimeter (Agilent Technologies, Mulgrave, Australia), at a wave length of 512 nm. Different assays of internalization were performed using different entities for internalization:

- CI VP1 GFP were IBs of bacterial origin and they are plotted as circles in FIG. 5.
- CI T22-GFP-H6 were IBs of bacterial origin and they are plotted as triangles in FIG. 5
- CI art (synthetic IBs) without lipid T22-GFP-H6, plotted as squares in FIG. 5 (manufactured as in Example 1 but without steps 4-5 and resuspending in step 6 the pellets of step 3 with a buffer containing functional protein of SEQ ID NO: 1)
- CI art (synthetic IBs) T22-GFP-H6 was prepared according to Example 1, plotted as rhombus.

CI VP1 GFP and CI T22-GFP-H6 bacterial IBs were obtained following the protocol disclosed by Unzueta et al., "Engineering tumor cell-targeting in nanoscale amyloidal materials", Nanotechnology-2017, vol. no.28, pp.: 015102. Briefly, protein of SEQ ID NO:1 was cloned into pET22b (Novagen) vector. E. coli strain Origami B Novagen) was transformed with the expression vector. IB production was carried out in a shake flask in Lysogenum Brooth (LB) medium (37 °C and 250 rpm) until reach an optical density at 550 nm of 0.5. Gene overexpression was induced (isopropyl β-D-1-thiogalactopyranoside IPTG 1 mM) and the subsequent protein deposition as IBs. Samples of cultures producing SEQ ID NO: 1 were harvested by centrifugation (150000 g for 15 min at 4 °C). Pellets were resuspended in lysis buffer and with lysozyme. Mechanical disruption was carried out after enzymatic digestion (French press, five rounds at 1200 psi). Samples were then frozen (-80°C) and after thawing IBs were washed while agitating. Several further steps of resuspension and centrifugation, together with freeze and thaw cycles were performed until no viable bacteria were present.

As can be deduced from FIG. 5, protein-lipid microparticles and nanoparticles internalized efficiently into cells. So do the nanoparticles of T22-GFP-H6 but in a minor extent. Therefore, protein-lipid particles (synthetic IBs) do really act as natural bacterial IBs.

Example 6. Cell viability in presence of protein-lipid microparticles and nanoparticles of the invention (synthetic IBs)

Toxicity of protein-lipid particles of the invention of Example 1 was determined at 24, 48 and 72 hours in CXCR4+ HeLa cells (ATCC® CCL-2™).

FIG. 6 is a graphic with the percentage of cell viability (% cell viability) of cells cultured with the presence of the following entities (as in Example 1):

- CI VP1 GFP, IBs of bacterial origin.
- CI T22-GFP-H6 IBs of bacterial origin.
- CI art (synthetic IBs) without lipid T22-GFP-H6 (manufactured as in Example 1 but without steps 4-5 and resuspending in step 6 the pellets of step 3 with a buffer containing functional protein of SEQ ID NO: 1)
- CI art (synthetic IBs) T22-GFP-H6 was prepared according to Example 1

The control (C) corresponds to the viability of the cells without the tested entities. It is used as 100% of cell viability.

The graphic shows that protein-lipid particles of Example 1 did not affect cell viability.

All these examples allow concluding that the particles of the invention, comprising a cluster of one or more types of assembled self-contained proteins configuring a tridimensional scaffold, are useful in the same way than natural bacterial inclusion bodies. They are stable, able to be internalized in tumor cells and they efficiently release any functional protein or even drug embedded within the cluster of assembled self-assembled protein molecules

Advantageously, drawbacks commonly associated (i.e. presence of bacterial components, non-homogeneity between batches, non-possibility of encapsulating proteins with post-translation modifications, and putative toxicity) with natural inclusion bodies are avoided with the protein nano- or microparticles according to the invention.

Example 7. Method for preparing protein microparticles and nanoparticles (synthetic IBs), using as pattern the fusion protein T22-GFP-H6 using ZnCl$_2$ as source of divalent cations

General procedure for this method of synthesis of protein nano- or microparticles of the invention is carried out according to the following detailed steps, herewith illustrated using protein T22-GFP-H6 (SEQ ID NO: 1):

1. Start from a purified protein sample in a specific storage buffer (generally sodium carbonate 166 mM with or without salt 333 mM) with a known concentration (generally above 2 mg/mL).

2. Start preparing a ZnCl$_2$ stock at a final concentration of 400 mM, final volume 50 mL in presence of distilled water.

3. After stock preparation, purified protein is diluted on its specific storage buffer till a final concentration of 2 mg/mL.

4. Separate the diluted protein in aliquots of 250 μL in eppendorfs.

5. Calculate the working proportion of 1:1 (ZnCl$_2$:Protein). At this step, the average or exact number of the histidine residues or of other amino acid residues that chelate with the divalent cation is needed. Below, there is exemplified the calculation considering the molecular weight of protein T22-GFP-H6 (SEQ ID NO: 1) and the six histidines comprised in the H6 tail segment of the fused protein

$$1\ \frac{mg}{mL} \cdot \frac{1000\ mL}{1\ L} \cdot \frac{1\ g}{1000\ mg} \cdot \frac{1\ mol}{30691.58\ g} = 3.26\ 10^{-5}\ \frac{mol}{L} = 0.0326\ \frac{mmol}{L}$$

$$x6\left(number\ of\ \frac{histidines}{protein}\right) \rightarrow 0.0326\ \frac{mmol}{L} \cdot 6 = 0.196\ mM$$

6. This 1:1 proportion corresponded to 0.196 mM of ZnCl$_2$ and is the one to be used to precipitate the diluted protein (2 mg/mL).

7. Proportions from 5:1 to 500:1 showed a clear aggregation tendency. In this example, the following were the selected proportions for precipitation and corresponding $ZnCl_2$ concentration in mM:

| Proportions (zinc chloride/protein) | ZnCl₂ concentration (mM) (1:1 = 0.196) |
|---|---|
| Wilde type | 0 |
| 5:1 | 0,98 |
| 10:1 | 1,96 |
| 20:1 | 3,92 |
| 30:1 | 5,88 |
| 40:1 | 7,84 |
| 50:1 | 9,8 |
| 60:1 | 11,76 |
| 70:1 | 13,72 |
| 100:1 | 19,6 |
| 150:1 | 29,4 |

8. Specific volume of $ZnCl_2$ was added depending on the final mM concentration of each proportion, the final volume (250 $\mu$L) and stock concentration 400 mM using the following equation:

$$V_i = \frac{V_f \cdot c_f}{c_i} = \frac{250\ \mu L \cdot ZnCl_2(mM)}{400\ mM}$$

9. Mixture of the protein and the salt was vortexed after adding $ZnCl_2$.

10. Precipitation of the protein (assembly of self-contained proteins) was seen as a formation of a mucus.

11. Mixtures were let for precipitation by waiting for 10 min at room temperature.

12. All samples at 15.000g at 4°C for 15 min were centrifuged.

13. Soluble from insoluble fraction were separated, this insoluble sample (pellets) corresponding to the protein nano- or microparticles (artificial IBs).

14. Protein concentration in the supernatant was quantified by Bradford assay.

15. By using the wild type as control (soluble protein sample without $ZnCl_2$ addition), the amount of formed precipitate in mg or in % was calculated.

[i.e. If the quantification of the soluble fraction is nearly 0 of a specific proportion and the wild type concentration is nearly 2 mg/mL, then a precipitation of 100% was considered and the precipitate was practically 2 mg/mL (magnitude corresponding to a soluble format). In % by weight or in weight of protein, the values were obtained by multiplying 2 mg/mL of the precipitate to the used volume (250 $\mu$L~ 0.250 mL) obtaining 0.5 mg or the corresponding % in respect of the wild type control.]

16. After Bradford calculation, the % of precipitate in respect of the used proportion of $ZnCl_2$ was plotted in a graphic to extrapolate precipitation if needed in other proportions.

17. Additionally, protein precipitation could be seen by means of the fluorescence or non-fluorescent of the pellets after centrifugation.

18. Proportions 100 and 150 induced 100 % of precipitation at this specific conditions.

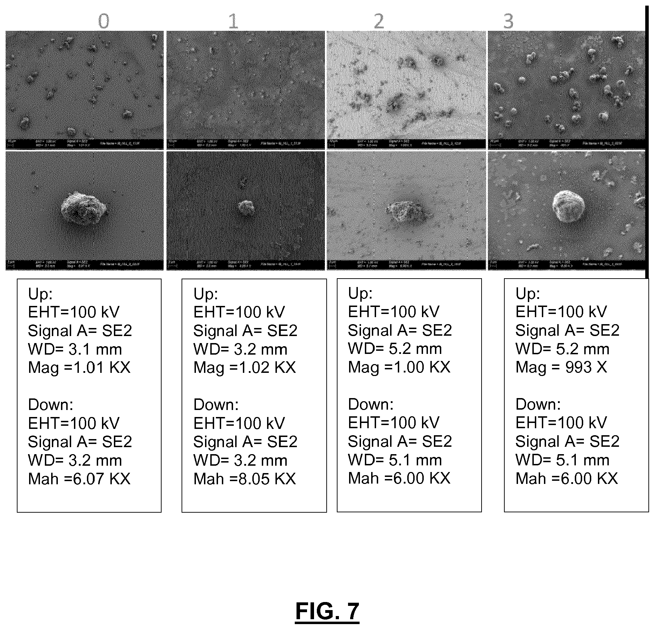

Protein nano and microparticles obtained following the above method can be seen in FESEM images of FIG. 7. This FIG. 7 includes images of several protein microparticles obtained by assembly of the fused protein T22-GFP-H6 with $ZnCl_2$ at different salt:protein molecular proportions. In images 1, 2 and 3 there are depicted the particles obtained using, respectively the molecular (or mol) proportions 40:1, 100:1 and 150:1 of salt:protein. Image 0 shows the image of a natural inclusion body produced directly in bacteria according to Unzueta et al, Nanotechnology 2017 (*supra*).

As can be seen in FIG. 7, with the method of the invention discrete protein particles resembling natural inclusions bodies were obtained. For this particular protein and salt of divalent cation, a proportion of moles of salt: moles of protein of 150:1 allow obtaining particle sizes similar to that of the natural bacterial inclusion body.

These data allow concluding that a method according to the invention is a good, reproducible and working method to obtain artificial inclusion bodies. In particular, that method comprising the steps of:

(a) mixing in a recipient one or more types of proteins in a polar solvent;
(b) adding to the mixture of step (a) a solution of salts of divalent cations at a final salt concentration of salts in the mixture allowing precipitation of the one or more proteins;
(c) isolating the precipitated one or more proteins from the solvent in which they were precipitated, which are protein nano- or microparticles comprising a cluster of assembled self-contained proteins, and optionally resuspending them in a fresh buffered solvent.

For the analysis of soluble protein delivery of the particles obtained using the method of divalent cations, artificial IBs in pellet format were resuspended in their corresponding buffers at a final concentration of 0.1 mg/mL and incubated without agitation at 37°C (physiological temperature). Aliquots of 150 $\mu$l were taken at defined times (from 0 to 10 days) and centrifuged for 15 min, 4°C at 15000g. Soluble and insoluble fraction were separated and further processed by Tris-Glycine eXtended (TGX) Stain-Free electrophoretic gels and Western Blot. In all cases, isolated insoluble fractions were then resuspended in their respective buffer at the same final volume than their soluble counterpart for comparison.

At this assayed conditions (pH around 7), the solubility (release) of the particles at day 10 was of 30% w/w of the proteins in the soluble fraction and 70% w/w in the pellet (insoluble fraction).

Measured after 15 days when implanted subcutaneously, artificial IBs of this example were able to release 50% of the assembled self-contained proteins. This means that used as delivery system, these artificial IBs do really were effective and entered systemic route even when applied as an implant, although they are delivery systems with a slow release profile.

**Citation List**

- Neubauer, et al., "Protein inclusion bodies in recombinant bacteria", 237-292, Springer- 2006.
- Garcia-Fruitos, E. et al. "Aggregation as bacterial inclusion bodies does not imply inactivation of enzymes and fluorescent proteins",Microbial cell factories2005, vol. no. 4, 27, doi:10.1186/1475-2859-4-27.
- Gonzalez-Montalban, N., et al., "Recombinant protein solubility - does more mean better?", Nature biotechnology 2007, vol. no. 25, pp.:718-720, doi:10.1038/nbt0707-718.
- Lopez-Laguna et al., "Assembly of histidine-rich protein materials controlled through divalent cations", Acta Biomaterialia 2019, vol. no. 83, pp.:257-264.
- Unzueta et al., "Engineering tumor cell-targeting in nanoscale amyloidal materials", Nanotechnology-2017, vol. no.28, pp.: 015102

**Claims**

1. Protein nano- or microparticle comprising a cluster of one or more types of assembled self-contained protein, wherein the micro/nanoparticle:

   - has a size, measured as hydrodynamic diameter, from 50 nanometers (nm) to 50 micrometers ($\mu$m);
   - is mechanically stable, which means that the cluster of self-contained proteins remains structured when submitted at sonication conditions including 5 rounds of 40 seconds; 0.5 of pulse on; 0.5 of pulse off and a wave width of 10 % in a high intensity sonicator Branson sonifier 450,with 3 mm-diameter titanium probe;
   - is in the form of a precipitated pellet in aqueous media, when centrifuged at 15.000 g at a temperature from 4 °C to 30 °C; and
   - it releases an amount of assembled self-contained proteins lower than 50 % by weight in relation to the total weight of assembled self-contained protein within 24 hours and when resuspended in an aqueous media at physiological temperature.

2. The protein nano- or microparticle according to claim 1, further comprising one or more salts of divalent cations.

3. The protein nano- or microparticle according to claim 2, wherein the divalent cations are selected from the group consisting $Be^{2+}$, $Mg^{2+}$, $Ca^{2+}$, $Sr^{2+}$, $Ba^{2+}$, $Ra^{2+}Zn^{2+}$, $Cu^{2+}$, $Ni^{2+}$, and combinations thereof.

4. The protein nano- or microparticle according to any one of claims 1-3, wherein the self-contained proteins are therapeutic proteins, said therapeutic proteins optionally covalently linked and/or conjugated to one or more additional different therapeutic agent.

5. The protein nano- or microparticle according to any one of claims 1-3, which is a protein-lipid nano- or microparticle that comprises a cluster of assembled self-contained proteins and one or more types of lipids assembled with the self-contained proteins.

6. The protein-lipid nano- or microparticle according to claim 5, wherein:

   - the self-contained proteins are denatured proteins and together with the assembled lipids configure a tridimensional scaffold; and

- the particle further comprises one or more functional proteins disposed within the tridimensional scaffold or adhered thereto.

7. The protein-lipid nano- or microparticle according to any one of claims 5-6, wherein the lipids are selected from the group consisting of fatty acids, glycerophospholipids, sterols, sphingolilpids, and combinations thereof.

8. The protein-lipid nano- or microparticle according to any ones of claims 5-7, wherein the one or more functional proteins are therapeutic proteins, optionally covalently linked and/or conjugated to a one or more additional different therapeutic agent.

9. Method for the synthesis of a protein nano- or microparticle as defined in any of claims 1 to 8, wherein the method comprises the following steps:

(a) mixing in a recipient one or more types of proteins in a polar solvent;
(b) submitting the mixture of step (a) to protein assembly conditions to obtain a protein-nano- or microparticle comprising a cluster of assembled self-contained proteins; and
(c) isolating the nano- or microparticle.

10. The method according to claim 9, wherein in step (b) the protein assembly conditions comprise the addition of salts to the mixture of step (a) at a final salt concentration of salts in the mixture allowing precipitation of the one or more proteins, and/or applying a protein-denaturation temperature.

11. The method according to any one of claims 9-10, wherein the method comprises the following steps:

(a) mixing in a recipient one or more types of proteins in a polar solvent;
(b) adding to the mixture of step (a) a solution of salts of divalent cations at a final salt concentration of salts in the mixture allowing precipitation of the one or more proteins;
(b) isolating the precipitated one or more proteins from the solvent in which they were precipitated, and optionally resuspending them in a fresh buffered solvent.

12. The method according to any one of claims 9-10, which is a method for the synthesis of a protein-lipid nano- or microparticle comprising the steps of:

(a) mixing in a recipient one or more types of proteins in a polar solvent;
(b) adding to the mixture of step (a) a solution of salts at a protein-denaturation salt concentration while and/or submitting the mixture to protein-denaturation temperature allowing precipitation of the one or more denatured proteins;
(c) isolating the precipitated one or more proteins from the solvent in which they were precipitated, and resuspending them in a fresh buffered solvent, said step of resuspending with a fresh solvent comprising:

(c.1) Mixing the precipitated protein with one or more lipids dissolved in an organic solvent and wherein the ratio of the amount of protein and lipids is from 0.8:1 to 1:0.8;
(c.2) removing the organic solvent to obtain a dry film of denatured proteins and lipids
(c.3) suspending the dry film of step (c.2) with a buffered composition, optionally comprising one or more functional proteins, while agitating the mixture under a controlled temperature from 4°C to 8 °C to obtain a protein-lipid nano- or microparticle comprising a cluster of assembled self-contained proteins and one or more types of lipids assembled with the self-contained proteins, said cluster optionally comprising one or more functional proteins embedded and/or adsorbed within the cluster of assembled self-contained proteins and lipids;
(c.4) separate the protein-lipid nano- and/or microparticle from the remaining buffered composition.

13. A protein nano- or microparticle as defined in any of claims 1-8, for use as a medicament.

14. The protein nano- or microparticle for use according to claim 13, which is for use in the treatment of a disease selected from the group consisting of cancer, an immune disease, neurodegenerative disease, and combinations thereof.

15. A pharmaceutical composition comprising a therapeutically effective amount of the protein a nano- or microparticle as defined any of claims 1-8, together with pharmaceutically acceptable excipients or carriers.

**FIG. 1**

(A)

EHT= 100 kV      Signal=SE2
WD= 3.3 mm      Mag= 2.56 KX

(B)

EHT= 100 kV      Signal=SE2
WD= 3.3 mm      Mag= 68.53 KX

## FIG. 2

**FIG. 3**

**FIG. 4**

**FIG. 5**

**FIG. 6**

| Up: | Up: | Up: | Up: |
|---|---|---|---|
| EHT=100 kV | EHT=100 kV | EHT=100 kV | EHT=100 kV |
| Signal A= SE2 | Signal A= SE2 | Signal A= SE2 | Signal A= SE2 |
| WD= 3.1 mm | WD= 3.2 mm | WD= 5.2 mm | WD= 5.2 mm |
| Mag =1.01 KX | Mag =1.02 KX | Mag =1.00 KX | Mag = 993 X |
| | | | |
| Down: | Down: | Down: | Down: |
| EHT=100 kV | EHT=100 kV | EHT=100 kV | EHT=100 kV |
| Signal A= SE2 | Signal A= SE2 | Signal A= SE2 | Signal A= SE2 |
| WD= 3.2 mm | WD= 3.2 mm | WD= 5.1 mm | WD= 5.1 mm |
| Mah =6.07 KX | Mah =8.05 KX | Mah =6.00 KX | Mah =6.00 KX |

## FIG. 7

Europäisches
Patentamt

European
Patent Office

Office européen
des brevets

# EUROPEAN SEARCH REPORT

Application Number

EP 19 38 2271

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | HÈCTOR LÓPEZ-LAGUNA ET AL: "Assembly of histidine-rich protein materials controlled through divalent cations", ACTA BIOMATERIALIA, vol. 83, 24 October 2018 (2018-10-24), pages 257-264, XP055623187, AMSTERDAM, NL ISSN: 1742-7061, DOI: 10.1016/j.actbio.2018.10.030 * abstract; figures 1,2,3,5 * * page 257, last paragraph - page 259, last paragraph * * page 261, last paragraph - page 262, last paragraph * | 1-4,9-15 | INV. B82Y5/00 |
| X | WO 2014/140023 A1 (AGRICULTURE AND FOOD DEV AUTHORITY TEAGASC [IE]) 18 September 2014 (2014-09-18) * claims 1,4,8,11,12,; examples 1,2 * | 1-4,9-11 | |
| X | EP 2 476 441 A1 (UNIV BARCELONA AUTONOMA [ES] ET AL.) 18 July 2012 (2012-07-18) * page 4 - page 7; claims 1-4,10-13; examples 1,2 * * page 10 - page 11 * * page 13, paragraph 2 * * page 15 - page 16 * | 1,4-9, 13-15 | TECHNICAL FIELDS SEARCHED (IPC) B82Y |

-/--

The present search report has been drawn up for all claims

| Place of search | Date of completion of the search | Examiner |
|---|---|---|
| The Hague | 19 September 2019 | Oderwald, Harald |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 3

Europäisches
Patentamt

European
Patent Office

Office européen
des brevets

**EUROPEAN SEARCH REPORT**

Application Number

EP 19 38 2271

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DILEK SALAM ET AL: "Preparation of high protein micro-particles using two-step emulsification", FOOD HYDROCOLLOIDS, ELSEVIER BV, NL, vol. 25, no. 5, 28 October 2010 (2010-10-28), pages 1139-1148, XP028163259, ISSN: 0268-005X, DOI: 10.1016/J.FOODHYD.2010.10.011 [retrieved on 2010-11-11] * abstract; figures 1,3 * * page 1140, last paragraph - page 1141, paragraph 4 * | 1,4,5, 7-10 | |
| X | FABIÁN RUEDA ET AL: "Structural and functional features of self-assembling protein nanoparticles produced in endotoxin-free Escherichia coli", MICROBIAL CELL FACTORIES, vol. 15, no. 1, 8 April 2016 (2016-04-08), XP055621655, DOI: 10.1186/s12934-016-0457-z * abstract; figure 1 * * page 2 - page 3 * | 1,4,9, 13-15 | |
| X | WO 2018/202921 A2 (UNIV AUTÒNOMA DE BARCELONA [ES] ET AL.) 8 November 2018 (2018-11-08) * examples 1-3,5,6,7,10,11 * | 1,4,9, 13-15 | TECHNICAL FIELDS SEARCHED (IPC) |

-/--

The present search report has been drawn up for all claims

| Place of search | Date of completion of the search | Examiner |
|---|---|---|
| The Hague | 19 September 2019 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 19 38 2271

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | UNZUETA UGUTZ ET AL: "Non-amyloidogenic peptide tags for the regulatable self-assembling of protein-only nanoparticles", BIOMATERIALS, vol. 33, no. 33, 2012, pages 8714-8722, XP028938886, ISSN: 0142-9612, DOI: 10.1016/J.BIOMATERIALS.2012.08.033 * abstract; figure 1; tables 1,2 * * page 8715 * | 1,4,9, 10,13 | |
| X | MARÍA VIRTUDES CÉSPEDES ET AL: "Selective depletion of metastatic stem cells as therapy for human colorectal cancer", EMBO MOLECULAR MEDICINE (ONLINE), vol. 10, no. 10, 9 October 2018 (2018-10-09), page e8772, XP055518166, DE ISSN: 1757-4684, DOI: 10.15252/emmm.201708772 * abstract * * page 2, paragraph 2 * * page 15, left-hand column, last paragraph - right-hand column, paragraph one * | 1,4,9, 10,13 | |
| X | ZOU LIQIANG ET AL: "Encapsulation of protein nanoparticles within alginate microparticles: Impact of pH and ionic strength on functional performance", JOURNAL OF FOOD ENGINEERING, BARKING, ESSEX, GB, vol. 178, 16 January 2016 (2016-01-16), pages 81-89, XP029431929, ISSN: 0260-8774, DOI: 10.1016/J.JFOODENG.2016.01.010 * page 82 - page 83; figure 1a * | 1,4,9,10 | TECHNICAL FIELDS SEARCHED (IPC) |

The present search report has been drawn up for all claims

| Place of search | Date of completion of the search | Examiner |
|---|---|---|
| The Hague | 19 September 2019 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 38 2271

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2019

| Patent document cited in search report | | Publication date | Patent family member(s) | | Publication date |
|---|---|---|---|---|---|
| WO 2014140023 | A1 | 18-09-2014 | NONE | | |
| EP 2476441 | A1 | 18-07-2012 | AU | 2012206533 A1 | 01-08-2013 |
| | | | CN | 103501818 A | 08-01-2014 |
| | | | DK | 2663336 T3 | 27-11-2017 |
| | | | EP | 2476441 A1 | 18-07-2012 |
| | | | EP | 2663336 A1 | 20-11-2013 |
| | | | ES | 2646390 T3 | 13-12-2017 |
| | | | IL | 227442 A | 31-05-2018 |
| | | | JP | 2014508515 A | 10-04-2014 |
| | | | PT | 2663336 T | 15-11-2017 |
| | | | US | 2014094404 A1 | 03-04-2014 |
| | | | US | 2017209590 A1 | 27-07-2017 |
| | | | US | 2019183968 A1 | 20-06-2019 |
| | | | WO | 2012095527 A1 | 19-07-2012 |
| WO 2018202921 | A2 | 08-11-2018 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NEUBAUER et al.** Protein inclusion bodies in recombinant bacteria. Springer, 2006, 237-292
- **GARCIA-FRUITOS, E. et al.** Aggregation as bacterial inclusion bodies does not imply inactivation of enzymes and fluorescent proteins. *Microbial cell factories,* 2005, 27
- **GONZALEZ-MONTALBAN, N. et al.** Recombinant protein solubility - does more mean better?. *Nature biotechnology,* 2007, 718-720
- **RINAS, U. et al.** Bacterial Inclusion Bodies: Discovering Their Better Half. *Trends in biochemical sciences,* 2017
- **DE MARCO, A.** Bacterial inclusion bodies are industrially exploitable amyloids. *FEMS microbiology reviews,* 2018
- **LOPEZ-LAGUNA et al.** Assembly of histidine-rich protein materials controlled through divalent cations. *Acta Biomaterialia,* 2019, 257-264
- **LÓPEZ-LAGUNA et al.** Assembly of histidine-rich protein materials controlled through divalent cations. *Acta Biomaterialia,* 2019, 257-264
- **UNZUETA et al.** Engineering tumor cell-targeting in nanoscale amyloidal materials. *Nanotechnology,* 2017, (28), 015102
- **UNZUETA et al.** *Nanotechnology,* 2017
- **GARCIA-FRUITOS, E. et al.** Aggregation as bacterial inclusion bodies does not imply inactivation of enzymes and fluorescent proteins. *Microbial cell factories,* 2005, (4), 27
- **GONZALEZ-MONTALBAN, N. et al.** Recombinant protein solubility - does more mean better?. *Nature biotechnology,* 2007, (25), 718-720
- **LOPEZ-LAGUNA et al.** Assembly of histidine-rich protein materials controlled through divalent cations. *Acta Biomaterialia,* 2019, (83), 257-264

SEQUENCE LISTING

<110>   UNIVERSITAT AUTÒNOMA DE BARCELONA

<120>   Protein nano-or microparticles as artificial inclusion bodies

<130>   T-2019/021

<160>   1

<170>   PatentIn version 3.5

<210>   1
<211>   269
<212>   PRT
<213>   Artificial

<220>
<223>   Fusion protein

<400>   1

Met Arg Arg Trp Cys Tyr Arg Lys Cys Tyr Lys Gly Tyr Cys Tyr Arg
1               5                   10                  15

Lys Cys Arg Gly Gly Ser Ser Arg Ser Ser Ser Lys Gly Glu Glu Leu
            20                  25                  30

Phe Thr Gly Val Val Pro Ile Leu Val Glu Leu Asp Gly Asp Val Asn
            35                  40                  45

Gly His Lys Phe Ser Val Ser Gly Glu Gly Glu Gly Asp Ala Thr Tyr
        50                  55                  60

Gly Lys Leu Thr Leu Lys Phe Ile Cys Thr Thr Gly Lys Leu Pro Val
65                  70                  75                  80

Pro Trp Pro Thr Leu Val Thr Thr Leu Thr Tyr Gly Val Gln Cys Phe
                85                  90                  95

Ser Arg Tyr Pro Asp His Met Lys Arg His Asp Phe Phe Lys Ser Ala
            100                 105                 110

Met Pro Glu Gly Tyr Val Gln Glu Arg Thr Ile Ser Phe Lys Asp Asp
            115                 120                 125

Gly Asn Tyr Lys Thr Arg Ala Glu Val Lys Phe Glu Gly Asp Thr Leu
        130                 135                 140

Val Asn Arg Ile Glu Leu Lys Gly Ile Asp Phe Lys Glu Asp Gly Asn
145                 150                 155                 160

Ile Leu Gly His Lys Leu Glu Tyr Asn Tyr Asn Ser His Asn Val Tyr

```
                        165                    170                        175


        Ile Thr Ala Asp Lys Gln Lys Asn Gly Ile Lys Ala Asn Phe Lys Ile
                    180                 185                 190


        Arg His Asn Ile Glu Asp Gly Ser Val Gln Leu Ala Asp His Tyr Gln
                    195                 200                 205


        Gln Asn Thr Pro Ile Gly Asp Gly Pro Val Leu Leu Pro Asp Asn His
            210                 215                 220


        Tyr Leu Ser Thr Gln Ser Ala Leu Ser Lys Asp Pro Asn Glu Lys Arg
        225                 230                 235                 240


        Asp His Met Val Leu Leu Glu Phe Val Thr Ala Ala Gly Ile Thr His
                    245                 250                 255


        Gly Met Asp Glu Leu Tyr Lys His His His His His
                    260                 265
```